# EUROPEAN PATENT SPECIFICATION

(11) **EP 2 236 510 B1**
(45) Date of publication and mention of the grant of the patent: **30.04.2014**
(21) Application number: 08867602.8
(22) Date of filing: 12.12.2008
(51) Int. Cl.: C07D 407/12

(54) **PROCESS FOR ISOLATION OF A MIXTURE OF RRRS AND SSSR CONFIGURATIONS OF NEBIVOLOL INTERMEDIATES**
VERFAHREN ZUR ISOLIERUNG EINER MISCHUNG VON RRRS- UND SSSR-KONFIGURATIONEN VON NEBIVOLOLZWISCHENPRODUKTEN
PROCÉDÉ D'ISOLEMENT D'UN MÉLANGE DES CONFIGURATIONS RRRS ET SSSR D'INTERMÉDIAIRES DU NÉBIVOLOL

(30) Priority: 21.12.2007 CN 200710172796
(43) Date of publication of application: 06.10.2010
(73) Proprietor: Shanghai Modern Pharmaceutical Co., Ltd., Shanghai 200137 (CN); Shanghai Institute of Pharmaceutical Industry, Shanghai 200040 (CN)
(72) Inventor: WANG, Guoping, Shanghai 200137 (CN); CHEN, Xudong, Shanghai 200137 (CN); HOU, Jian, Shanghai 200137 (CN)
(74) Representative: Hannke, Christian
(86) International application number: PCT/CN2008/073492
(87) International publication number: WO 2009/082913

(56) References cited:
- WO-A1-2006/016376
- WO-A1-2007/083318
- WO-A1-2007/083318

## Description

### Field of the Invention

The present invention relates to a process for preparing a mixture of isomers of one compound, specifically relates to the process for preparation of a mixture of RRRS and SSSR configurations of Nebivolol intermediates.

### Background of the Invention

As shown below, the structure of Nebivolol contains four chiral carbon atoms and there are 16 stereoisomers in theory, but only 10 exist considering symmetry factors.

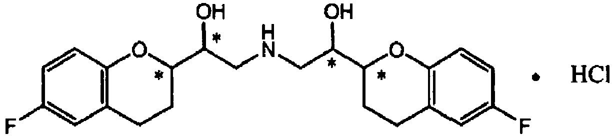

Wherein, d-nebivolol (RRRS) and l-nebivolol (SSSR) are β-receptor blockers, with anti-hypertensive effect.

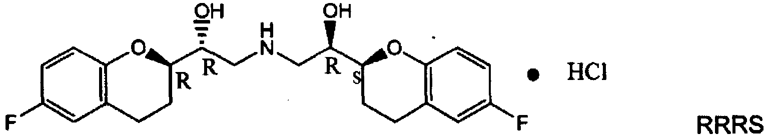

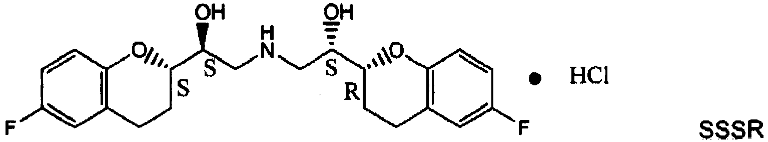

Patents EP145067 and EP334429 described the preparation of Nebivolol and related compounds. The synthetic route is as follows:

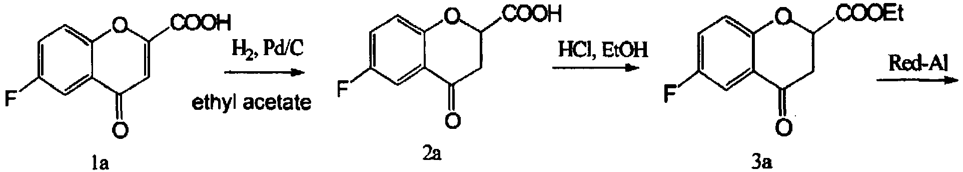

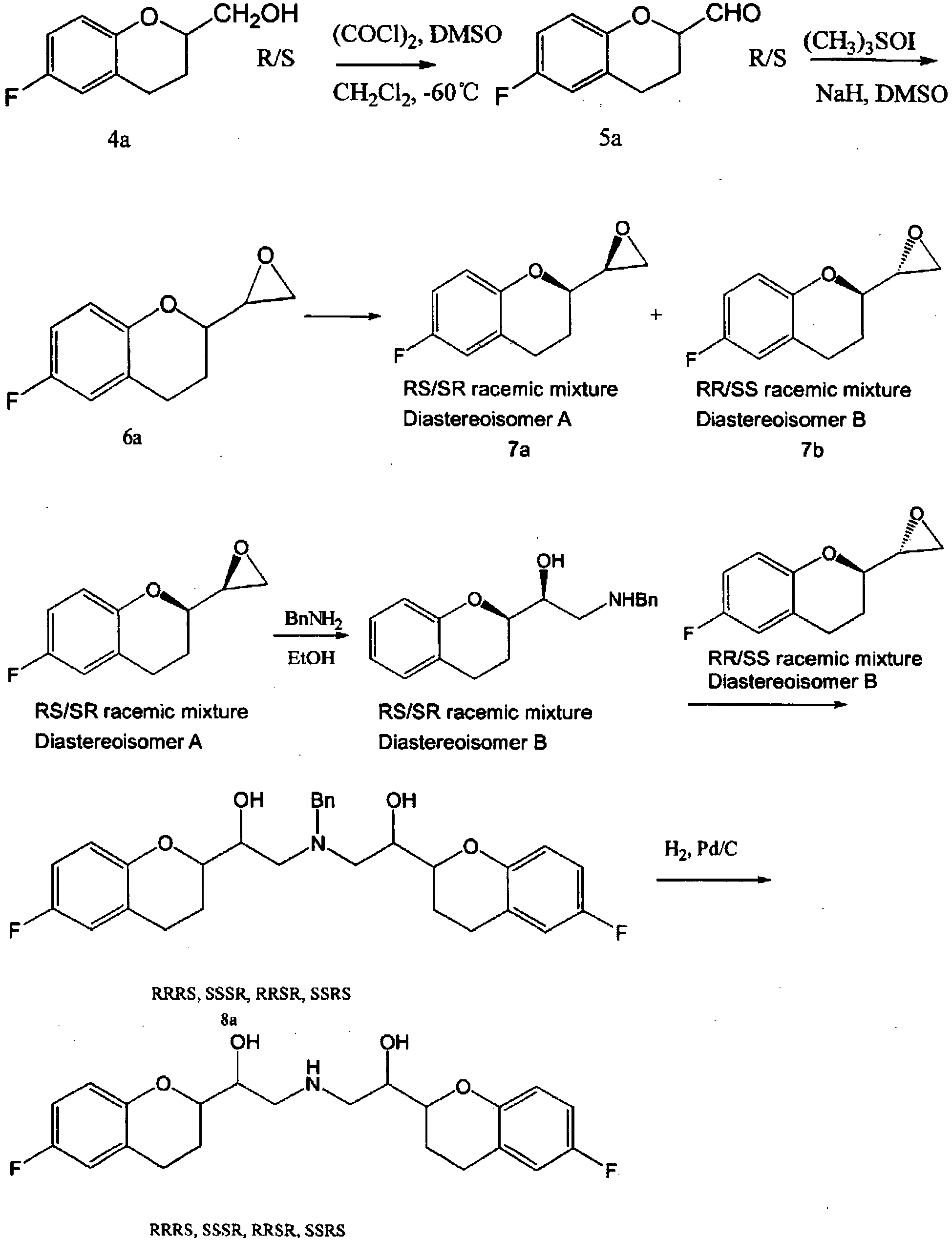

In those patents, except for 1a, 2a, 3a, 4a and 5a, there is no example which specifically describes the process of preparation of those compounds, and the separation and purification process of 7a and 7a is not described either. Yet these compounds can be prepared according to similar methods. According to the patents, the product obtained is the mixture of Nebivolol (RRRS, SSSR, RRSR, SSRS). US5759580 reported the method of fractional crystallization to separate the mixture but with quite low yield (6.6%). Therefore it is considered that, 8a is the most critical intermediate for preparation of Nebivolol, not only because Nebivolol can be obtained from 8a by debenzylation, but also because the desired stereoisomer of Nebivolol can be directly obtained by controlling the stereo configuration of 8a.

According to the approach described above, the obtained 8a is the mixture of (RRRS, SSSR, RRSR, SSRS):

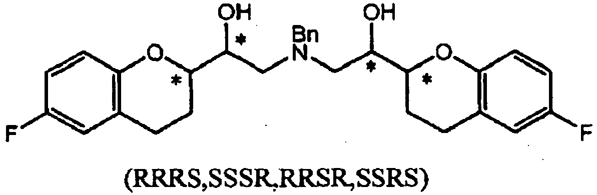

Wherein (RRRS, SSSR) are the desired intermediates:

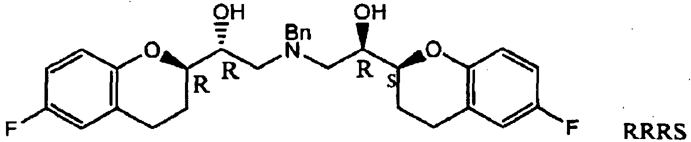

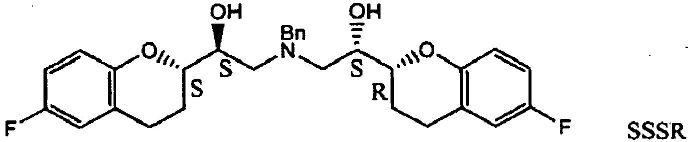

W02006016376 reported that 8a was converted into its hydrochloride salt, then after fractional recrystallization and dissociation, the desired stereoisomer was obtained. The disadvantages of this method were the long steps and low yield.

The improvement of this method was reported in WO2007083318, in which after 8a was produced, the solvent ethanol was evaporated, and the racemic mixture was dissolved in an ether solvent and then the desired crystals were isolated from that mixture. The solid product that could be obtained was recrystallized from isopropyl. But after being treated for two times, 2% of other isomers still remained, which could cause inconvenience to subsequent operations. Thus according to W02007083318 the desired compounds of formula (II) and (III) can be obtained by a complex process comprising cooling, seeding, partial removal of the solvent from the solution or by addition of an anti-solvent to the solution.

### Summary of the Invention

It is an object of the present invention to provide an improved simple and efficient processes for the preparation of the nebivolol intermediates of formula (II) and (III) and the separation out of a mixture of RRRS and SSSR configurations of Nebivolol intermediates, which solves the technical problems by overcoming the defects of inconvenient operations of the key steps and low separation efficiency in separation of RRRS, SSSR, RRSR and SSRS configurations of intermediate mixture, in the existing synthetic method of preparing the mixture of RRRS and SSSR configurations of Nebivolol.

The method of the present invention for preparing a mixture of RRRS and SSSR configurations of nebivolol intermediates represented by formula II and III respectively is optionally selected from one of the following two manners:
(1) To the alcohol solvent comprising the mixture of RRRS, SSSR, RRSR and SSRS configurations of nebivolol intermediates represented by formula I, is added the precipitation solvent; after heating, cooling down to precipitate crystals and filtrating, the product can be obtained;
(2) The mixture of RRRS, SSSR, RRSR and SSRS configurations of nebivolol intermediates represented by formula I is added into the mixed solvent of alcohol solvent and precipitation solvent; after heating, cooling down to precipitate crystals and filtrating, the product can be obtained.

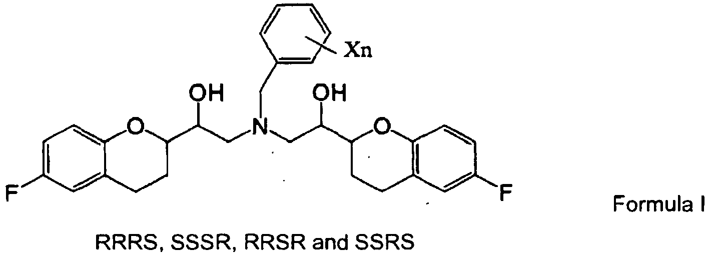

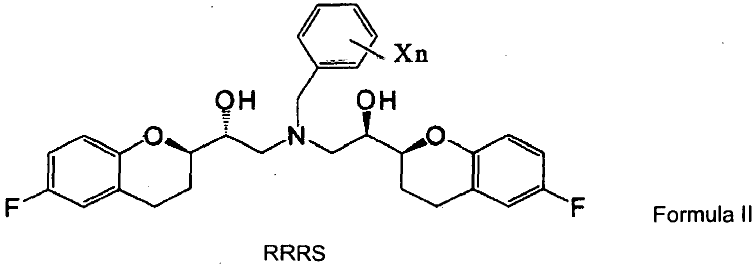

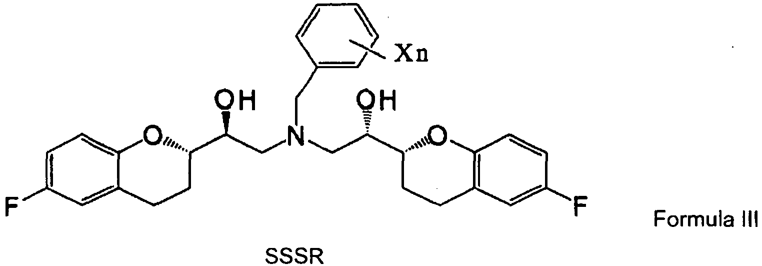

Wherein, X is H, C₁-C₆ alkyl or C₁-C₆ alkoxy, n is 1 -5 and wherein the said precipitation solvent is hydrocarbon solvents and/or ether solvents.

Wherein, the said alcohol solvent can be preferably one or more selected from group consisting of methanol, ethanol, propanol and isopropyl alcohol, more preferably is ethanol. The said precipitation solvent can be preferably C5~C9 hydrocarbon solvents or ether solvents, including cyclic hydrocarbon, preferably is n-hexane, n-heptane and/or cyclohexane, and more preferably is n-hexane. The said precipitation solvent can also be preferably the ether solvent, more preferably is isopropyl ether. The volume ratio of the said precipitation solvent and the said alcohol solvent can be preferably 1:2~4:1, more preferably is 2:1. The total amount of the said precipitation solvent and the said alcohol solvent is at least the amount that can solve the mixture of RRRS, SSSR, RRSR and SSRS configurations of nebivolol intermediates after heating, more preferably is 1 to 20 times of the quality of nebivolol intermediates mixture. The temperature of the said heating can be preferably 30~80°C, and more preferably is the reflux temperature of the solvents. The temperature of the said cooling can be preferably 0~50°C, more preferably is 10~15°C. The time period of the precipitation of crystals can be preferably 2-18 hours.

In one preferable example of the present invention, the reaction system which is prepared using alcohol solvent and contained the mixture of intermediates represented by formula IV (X = H) is cooled down to 50°C. Then hexane in double the volume of ethanol is added. After that, the reaction system is heated to reflux, and then cooled down to 10~15°C. After crystallizing for 4 hours and filtering, the mixture of RRRS and SSSR configurations of nebivolol intermediates represented by formulae IV and V respectively can be obtained.

In another preferable example of the present invention, the mixed solvent of alcohol and hexane (v/v = 1: 2) is added into the mixture of intermediates represented by formula IV (X = H). Then the solution is heated to reflux, and then cooled down to 10~15°C. After crystallizing for 4 hours and filtrating, the mixture of RRRS and SSSR configurations of nebivolol intermediates represented by formulae V and VI respectively can be obtained.

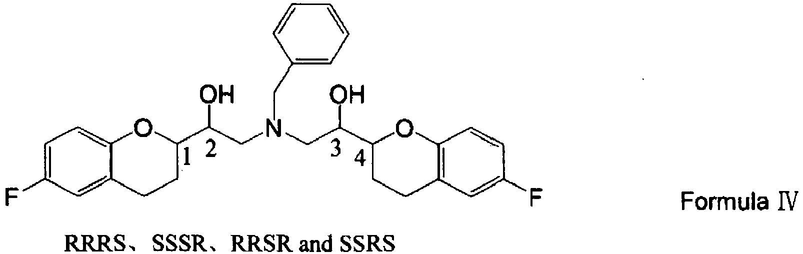

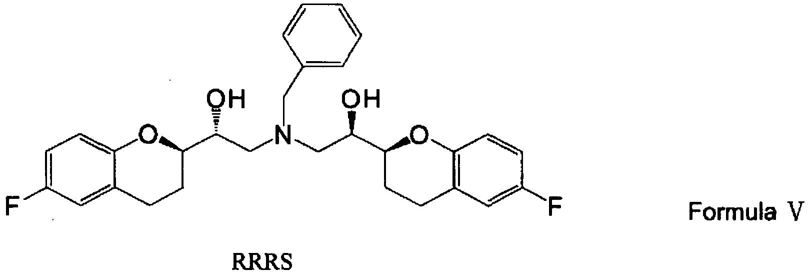

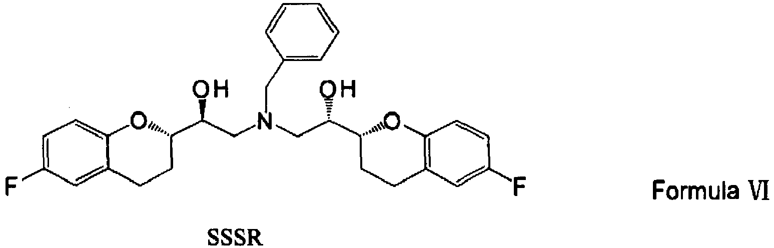

In the present invention, the mixture of RRRS and SSSR configurations of Nebivolol can be obtained from the mixture of RRRS and SSSR configurations of nebivolol intermediates represented by formulae II and III by debenzylation.

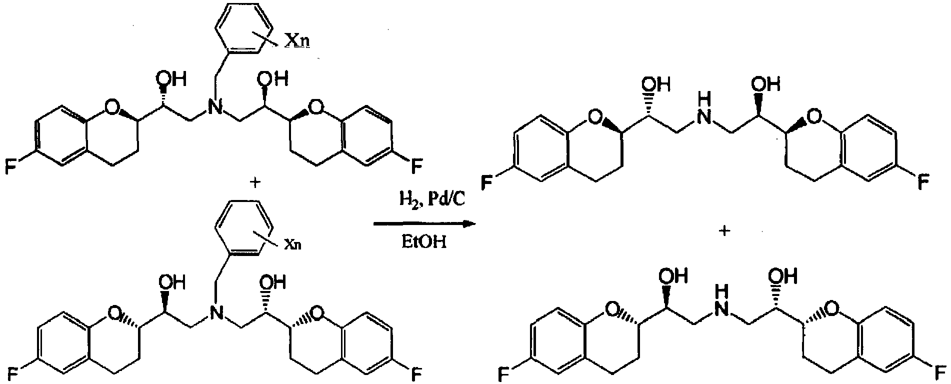

In the present invention, the preparation of the mixture of RRRS, SSSR, RRSR and SSRS configurations of nebivolol intermediates represented by formulae I can refer to the method described in EP145067 and EP334429. Other reagents and materials used in this invention are commercially available.

The advantages of the present invention are the simple operation, high separation efficiency, and convenience to be scaled up to industrial scale.

### Examples

The following examples provide a more detailed explanation of the present invention, but the present invention is not limited thereto.

### Reference example 1 Preparation of the mixture of RRRS, SSSR, RRSR and SSRS configurations of nebivolol intermediates (formulae I, X = hydrogen)

To a 2000ml reaction flask, was added successively 1000ml of absolute ethanol, 100g of (±) [1S*(R)]-6-fluoro-3,4-dihydro-2H-benzopyran-2-ethylene oxide (7b, ref: EP145067 and EP334429), and 185g of benzylamine. After refluxing for 6 hours, cooling down to 0°C, keeping warm for 4 hours, and filtering, the filter cake was obtained. 85g of (±) [1S*(R)]-6-fluoro-3,4-dihydro-α-[[(benzyl)amino] methyl]-2H-benzopyran-2-methanol was obtained by drying under 40°C.

To a 2000ml reaction flask, was added 500ml of absolute ethanol, 85g of (±) [1S*(R)]-6-fluoro-3,4-dihydro-α-[[(benzyl)amino]methyl]-2H-benzopyran-2-methanol, 70g of (±) [IS*(S)]-6-fluoro-3,4-dihydro-2H-benzopyran-2-ethylene oxide. After refluxing for 15 hours, cooling down to 30°C, alcohol solution (500ml) comprising the mixture of RRRS, SSSR, RRSR and SSRS configurations of α,α'-[benzyliminobis(methylene)] bis-[6-fluoro-3,4-dihydrogen -2H-benzopyran-2-methanol] (formula IV) was obtained.

### Example 1 Preparation of the mixture of RRRS and SSSR configurations of nebivolol intermediates (formula 1, X = H)

To the solution obtained in reference example 1, was added 250ml of n-hexane. After heating to 30°C, cooling down to 10~15°C, keeping warm for 4 hours for crystallization, and filtering, 67g of (±) [R*[S*[S*(S*)]]]α,α'-[benzyliminobis(methylene)]bis-[6-fluoro-3,4-dihydrogen-2H-benzopyran -2-methanol] (the mixture of compounds shown as formulae V and VI) was obtained (HPLC purity 99.2%).

### Example 2 Preparation of the mixture of RRRS and SSSR configurations of nebivolol intermediates (formula I, X = H)

To the mixture (formula IV, 160g) which was obtained by evaporating in vacuo and recovering the alcohol in the solution obtained in reference example 1, was added 160g of mixed solution (ethanol : isopropyl ether = 1 : 4). After heating to reflux, cooling down to 10~15°C, keeping warm for 18 hours for crystallization, and filtering, 68g of (±) [R*[S*[S*(S*)]]] α,α'-[benzyliminobis(methylene)]bis-[6-fluoro-3,4-dihydrogen-2H-benzopyran-2-methanol] (the mixture of compounds shown as formulae V and VI) was obtained (HPLC purity 99.6%).

### Example 3 Preparation of the mixture of RRRS and SSSR configurations of nebivolol intermediates (formula I, X = H)

To the solution obtained in reference example 1, was added 1000ml of n-hexane. After heating to reflux, cooling down to 10~15°C, keeping warm for 4 hours for crystallization, and filtering, 67g of (±) [R*[S*[S*(S*)]]]α,α'-[benzylimino bis(methylene)]bis-[6-fluoro-3,4-dihydrogen-2H-benzopyran-2-methanol] (the mixture of compounds shown as formulae V and VI) was obtained (HPLC purity 99.2%).

### Example 4 Preparation of the mixture of RRRS and SSSR configurations of nebivolol intermediates (formula I, X = H)

To the mixture (formula IV, 160g) which was obtained by evaporating in vacuo and recovering the alcohol in the solution obtained in reference example 1, was added 2318ml of mixed solution (ethanol : n-hexane = 1 : 2). After heating to reflux, cooling down to 10~15°C, keeping warm for 4 hours for crystallization, and filtering, 68g of (±)[R*[S*[S*(S*)]]]α,α'-[benzyliminobis(methylene)] bis-[6-fluoro-3,4-dihydrogen-2H-benzopyran-2-methanol] (the mixture of compounds shown as formulae V and VI) was obtained (HPLC purity 99.3%).

### Reference Example 2 Preparation of RRRS and SSSR configurations of Nebivolol

To a 1000ml hydrogenation reactor, was added 300ml of absolute ethanol, 67g of the product obtained from Example land 5g of 10% palladium carbon. After replacing air by inletting nitrogen, heating to 50°C, stopping inletting nitrogen, and starting inletting hydrogen for about 4 hours, the completion of the reaction is determined by TLC test. After removing the palladium carbon by filtration, the filtrate was evaporated in vacuo to 1/5 of the volume. The pH value was modulated to 2-3 by adding concentrated hydrochloric acid. Then after stirring for 1 hour, cooling, crystallizing, keeping warm at 0°C for 6 hours and filtering, the filter cake was washed with a small amount of ethanol to get 55g of crude product. 40g of RRRS and SSSR configurations of Nebivolol was obtained by recrystallization with absolute ethanol.

### Reference Example 3 Preparation of RRRS and SSSR configurations of Nebivolol hydrochloric acid

66g of the solid obtained from Example 2 was dissolved in 400ml of heating ethanol. After cooling down to 40°C, the pH value was modulated to 2-3 by adding concentrated hydrochloric acid. After stirring for 1 hour, cooling, crystallizing, keeping warm at 0°C for 6 hours and filtering, the filter cake was washed with a small amount of ethanol to get 68g of the product.

To a 2000ml hydrogenation reactor, was added 1200ml of absolute methanol, 68g of the product described above and 5g of 10% palladium carbon. After replacing air by inletting nitrogen, heating to 40°C, stopping inletting nitrogen and starting inletting hydrogen for about 4 hours, the completion of the reaction is determined by TLC test. After removing the palladium carbon by filtration, the filtrate was evaporated in vacuo to dryness. 39g of the mixture of RRRS and SSSR configurations of Nebivolol was obtained by recrystallization with absolute ethanol.

### Reference example 4 Preparation of the mixture of RRRS, SSSR, RRSR and SSRS configurations of nebivolol intermediates (formulae I, X = 4-methoxyl, n = 1)

To a 1000ml reaction flask, was added successively 500ml of ethanol, 100 g of (±) [1S*(R)]-6-fluoro-3,4-dihydro-2H-benzopyran-2-ethylene oxide (7b, ref: EP145067 and EP334429), and 170g of 4-methoxyl benzylamine. After refluxing for 24 hours, cooling down to 0°C, keeping warm for 4 hours and filtering, the filter cake was obtained. 94g of (±) [1S*(R)]-6-fluoro-3,4-dihydro-α-[[(4-methoxylbenzyl)amino]methyl]-2H-benzopyran-2-methanol was obtained by drying at 40°C.

To a 2000ml reaction flask, was added 300ml of methanol, 94g of (±) [1S*(R)]-6-fluoro-3,4-dihydro-α-[[(4-methoxylbenzyl)amino]methyl]-2H-benzopyran-2-methanol and 67g of (±) [1S*(S)]-6-fluoro-3,4-dihydro-2H-benzopyran-2-ethylene oxide. After heating to reflux for 15 hours and cooling down to 30°C, ethanol solution(300ml) comprising the mixture of RRRS, SSSR, RRSR and SSRS configurations of α,α'-[4-methoxybenzyliminobis(methylene)] bis-[6-fluoro-3,4-dihydrogen-2H-benzopyran-2-methanol] (formula IV) was obtained.

### Example 5 Preparation of the mixture of RRRS and SSSR configurations of nebivolol intermediates (formula I, X = 4-methoxyl)

To the solution obtained in reference example 4, was added 900ml of n-heptane. After heating to 50°C, cooling down to 0°C, keeping warm for 2 hours for crystallization and filtering, the mixture of RRRS and SSSR configurations of nebivolol intermediates (formula I, X = 4-methoxyl, 70g) was obtained (HPLC purity 99.6%).

### Example 6 Preparation of the mixture of RRRS and SSSR configurations of nebivolol intermediates (formula I, X = 4-methoxyl)

To the solid mixture (162g) which was obtained by evaporating in vacuo and recovering the alcohol in the solution obtained in reference example 4, was added 3240g of mixed solution (ethanol : cyclohexane = 1 : 3). After heating to 80°C, cooling down to 50°C, keeping warm for 18 hours for crystallization and filtering, the mixture of RRRS and SSSR configurations of nebivolol intermediates (formula I, X = 4-methoxyl, 77g) was obtained (HPLC purity 99.2%).

### Reference example 5 Preparation of the mixture of RRRS, SSSR, RRSR and SSRS configurations of nebivolol intermediates (formulae I, X = 4-methyl, n = 1)

To a 1000ml reaction flask, was added successively 500ml of isopropanol, 100g of (±) [1S*(R)]-6-fluoro-3,4-dihydro-2H-benzopyran-2-ethylene oxide and 170g of 4-methyl benzylamine. After refluxing for 6 hours, cooling down to 0°C, keeping warm for 4 hours and filtering, 97g of filter cake of (±)[1S*(R)]-6-fluoro-3,4-dihydro-α-[[(4-methylbenzyl) amino]methyl]-2H-benzopyran-2-methanol was obtained.

To a 2000ml reaction flask, was added 300ml of isopropanol, 97g of (±) [1S*(R)]-6-fluoro-3,4-dihydro-a-[[(4-methylbenzyl)amino]methyl]-2H-benzopyran-2-methanol and 67.5g of (±) [1S*(S)]-6-fluoro-3,4-dihydro-2H-benzopyran-2-ethylene oxide. After refluxing for 15 hours, cooling down to 50 °C, isopropanol solution (300ml) comprising the mixture of RRRS, SSSR, RRSR and SSRS configurations of α,α'-[4-methylbenzyliminobis(methylene)]bis -[6-fluoro-3,4-dihydrogen-2H-benzopyran-2- methanol] was obtained.

### Example 7 Preparation of the mixture of RRRS and SSSR configurations of nebivolol intermediates (formula I, X = 4-methyl, n = 1)

To the solution obtained in reference example 5, was added 600ml of petroleum ether. After heating to 70°C, cooling down to 15°C, keeping warm for 10 hours for crystallization, and filtering, the mixture of RRRS and SSSR configurations of α,α'-[4-methylbenzyliminobis(methylene)]bis-[6-fluoro-3,4-dihydrogen-2H-benzopyran-2- methanol] (75g) was obtained (HPLC purity 99.1%).

### Example 8 Preparation of the mixture of RRRS and SSSR configurations of nebivolol intermediates (formula I, X = 4-methyl, n = 1)

To the mixture (formula IV, 163 g) which was obtained by evaporating in vacuo and recovering the alcohol in the solution obtained in reference example 5, was added 1630g of mixed solution (ethanol : n-nonane = 1 : 1). After heating to reflux, cooling down to 10°C, keeping warm for 4 hours for crystallization and filtering, the mixture of RRRS and SSSR configurations of α,α'-[4-methylbenzylimino bis (methylene)] bis- [6-fluoro- 3,4- dihydrogen -2H- benzopyran -2-methanol] (76g) was obtained (HPLC purity 99.3%).

## Claims

1. A process for preparing a mixture of RRRS and SSSR configurations of nebivolol intermediates represented by formula II and III respectively, **characterized in that**: one of the following two manners is optionally selected:
(1) To the alcohol solvent comprising the mixture of RRRS, SSSR, RRSR and SSRS configurations of nebivolol intermediates represented by formula I, is added the precipitation solvent; after heating, cooling down to precipitate crystals and filtrating, the product can be obtained;
(2) The mixture of RRRS, SSSR, RRSR and SSRS configurations of nebivolol intermediates represented by formula I is added into the mixed solvent of alcohol solvent and precipitation solvent; after heating, cooling down to precipitate crystals and filtrating, the product can be obtained; 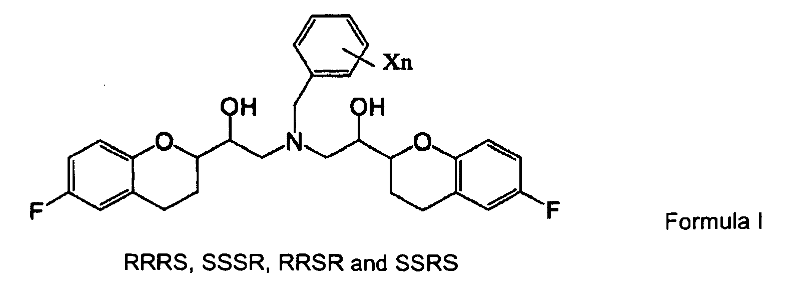 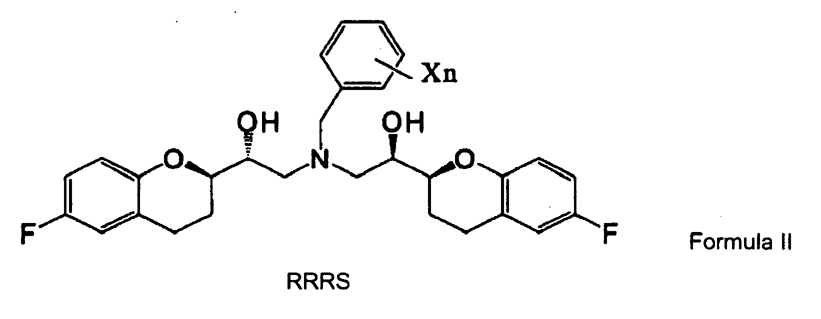 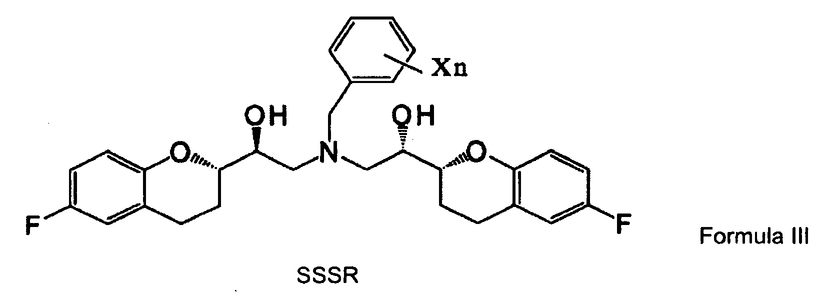
Wherein, X is H, C₁-C₆ alkyl or C₁-C₆ alkoxy, n is 1-5 and wherein the said precipitation solvent is hydrocarbon solvents and/or ether solvents.

2. The process according to claim 1, **characterized in that**: the said alcohol solvent is one or more selected from group consisting of methanol, ethanol, propanol and isopropyl alcohol.

3. The process according to claim 2, **characterized in that**: the said alcohol solvent is ethanol.

4. The process according to claim 1, **characterized in that**: the said hydrocarbon solvents is C₅ ~ C₉ hydrocarbon solvents; the said ether solvent is isopropyl ether.

5. The process according to claim 4, **characterized in that**: the said C₅ ~ C₉ hydrocarbon solvent is one or more selected from group consisting of n-hexane, n-heptane, petroleum ether and cyclohexane.

6. The process according to claim 5, **characterized in that**: the said C₅ ~ C₉ hydrocarbon solvent is n-hexane.

7. The process according to claim 1, **characterized in that**: the volume ratio of the said precipitation solvent and the said alcohol solvent is 1:2 - 4:1.

8. The process according to claim 7, **characterized in that**: the volume ratio of the said precipitation solvent and the said alcohol solvent is 2:1.

9. The process according to claim 1, **characterized in that**: the temperature of the said heating is 30 - 80°C.

10. The process according to claim 9, **characterized in that**: the temperature of the said heating is the reflux temperature of the solvents.

11. The process according to claim 1, **characterized in that**: the temperature of the said cooling is 0 - 50°C.

12. The process according to claim 11, **characterized in that**: the temperature of the said cooling is 10 - 15°C.

13. The process according to claim 1, **characterized in that**: the said time period of precipitation of crystals is 2 - 18 hours.

14. The process according to claim 1, **characterized in that**: to the alcohol solution that contains the mixture of RRRS, SSSR, RRSR and SSRS configurations of nebivolol intermediate represented by formula I, is added n-hexane in double the volume of ethanol, then the solution is heated to reflux and cooled down to 10 - 15°C, after crystallizing for 4 hours and filtering, the product can be obtained.

15. The process according to claim 1, **characterized in that**: the mixed solvent of alcohol and n-hexane with a v/v ratio of 1:2 is added into the mixture of RRRS, SSSR, RRSR and SSRS configurations of nebivolol intermediate represented by formula I, then the solution is heated to reflux, and cooled down to 10 ~ 15°C; after crystallizing for 4 hours and filtrating, the product can be obtained.

## Patentansprüche

1. Verfahren zur Herstellung einer Mischung aus RRRS und SSSR-Konfigurationen von Nebivolov-Zwischenprodukten gemäß jeweils Formel II and III, **dadurch gekennzeichnet, dass**: eine der beiden folgenden Arten optional ausgewählt wird:
(1) Zu einem Alkohol-Lösungsmittel, das die Mischung aus RRRS, SSSR, RRSR und SSRS-Konfigurationen von Nebivolov-Zwischenprodukten gemäß Formel I enthält, wird ein Fällungslösungsmittel gegeben; nach Erwärmen, Abkühlen zum Ausfällen von Kristallen und Filtern kann das Produkt erhalten werden;
(2) Die Mischung von RRRS, SSSR, RRSR und SSRS-Konfigurationen von Nebivolov-Zwischenprodukten gemäß Formel I wird in ein Lösungsmittelgemisch aus Alkohol-Lösungsmittel und Fällungslösungsmittel gegeben; nach Erwärmen, Abkühlen zum Ausfällen von Kristallen und Filtern kann das Produkt erhalten werden; 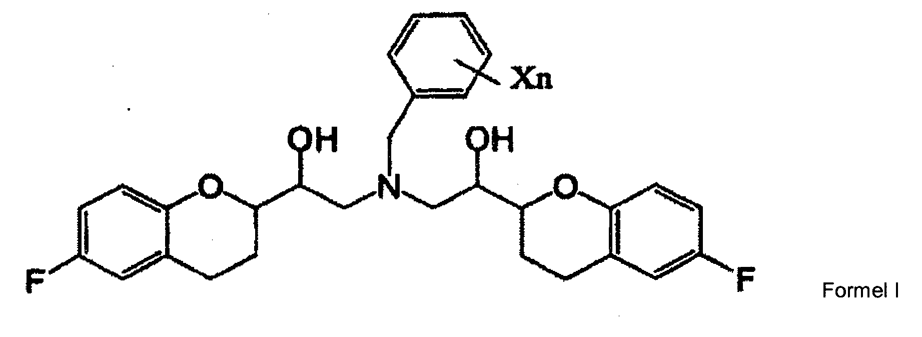 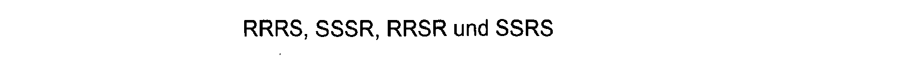 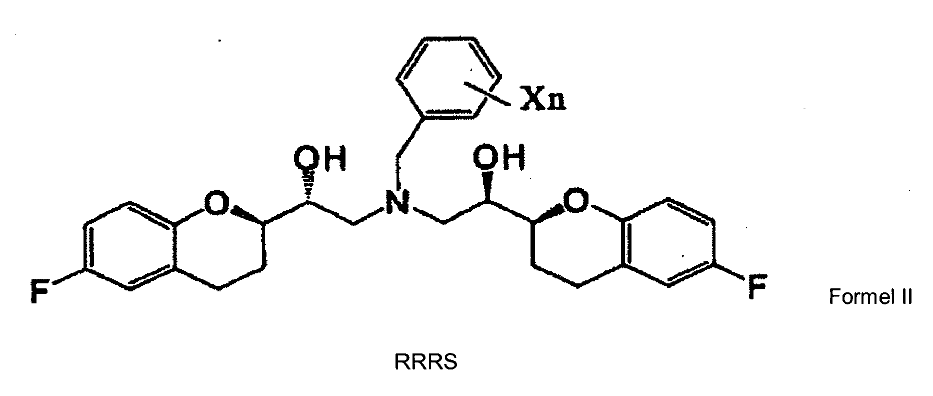 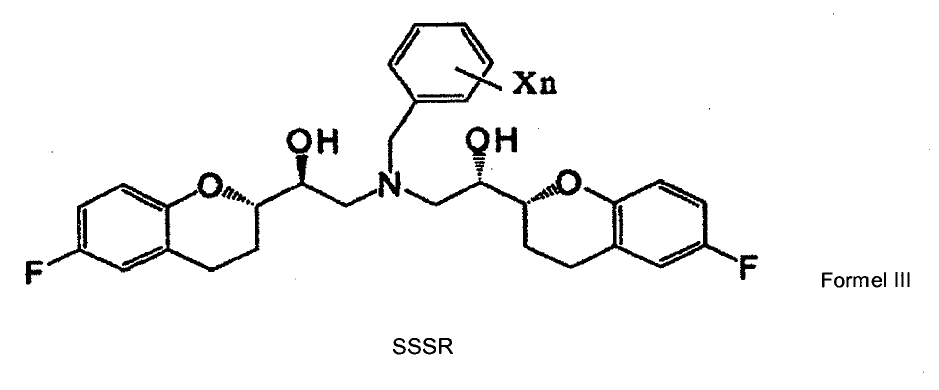
wobei X H, C1 - C6-alkyl oder C1 - C6-alkoxy ist, n 1 - 5 ist und wobei das Fällungslösungsmittel ein Kohlenwasserstoff-Lösungsmittel und/oder ein Ether-Lösungsmittel ist.

2. Verfahren gemäß Anspruch 1, **dadurch gekennzeichnet, dass** das Alkohol-Lösungsmittel eines ist oder mehrere sind, ausgewählt aus einer Gruppe bestehend aus Methanol, Ethanol, Propanol und Isopropylalkohol.

3. Verfahren nach Anspruch 2, **dadurch gekennzeichnet, dass** das Alkohol-Lösungsmittel Ethanol ist.

4. Verfahren gemäß Anspruch 1, **dadurch gekennzeichnet, dass** das Kohlenwasserstoff-Lösungsmittel ein C5 - C9-Kohlenwasserstoff-Lösungsmittel, das Ether-Lösungsmittel Isopropylether ist.

5. Verfahren gemäß Anspruch 4, **dadurch gekennzeichnet, dass** das C5 - C9-Kohlenwasserstoff-Lösungsmittel eines ist oder mehrere sind, ausgewählt aus einer Gruppe bestehend aus n-Hexan, n-Heptan, Petroleum-Ether und Cyclohexan.

6. Verfahren gemäß Anspruch 5, **dadurch gekennzeichnet, dass** das C5 - C9-Kohlenwasserstoff-Lösungsmittel n-Hexan ist.

7. Verfahren gemäß Anspruch 1, **dadurch gekennzeichnet, dass** das Volumenverhältnis des Fällungslösungsmittels und des Alkohol-Lösungsmittels 1 : 2 - 4 : 1 ist.

8. Verfahren gemäß Anspruch 7, **dadurch gekennzeichnet, dass** das Volumenverhältnis des Fällungslösungsmittels und des Alkohol-Lösungsmittels 2 : 1 ist.

9. Verfahren gemäß Anspruch 1, **dadurch gekennzeichnet, dass** die Temperatur des Erwärmens 30 - 80°C ist.

10. Verfahren gemäß Anspruch 9, **dadurch gekennzeichnet, dass** die Temperatur des Erwärmens die Rückflusstemperatur des Lösungsmittels ist.

11. Verfahren gemäß Anspruch 1, **dadurch gekennzeichnet, dass** die Temperatur des Kühlens 0 - 50°C ist.

12. Verfahren gemäß Anspruch 11, **dadurch gekennzeichnet, dass** die Temperatur des Kühlens 10 - 15°C ist.

13. Verfahren gemäß Anspruch 1, **dadurch gekennzeichnet, dass** der Zeitabschnitt des Kristallisierens 2 - 18 Stunden ist.

14. Verfahren gemäß Anspruch 1, **dadurch gekennzeichnet, dass** zu der Alkohollösung, die die Mischung von RRRS, SSSR, RRSR und SSRS-Konfigurationen von Nebivolov-Zwischenprodukten gemäß Formel I enthält, n-Hexan im doppelten Volumen vom Ethanol zugegeben wird, dann die Lösung zum Rückfluss erhitzt wird und auf 10 - 15°C abgekühlt wird und nach Kristallisieren für 4 Stunden und Filtrieren das Produkt erhalten werden kann.

15. Verfahren gemäß Anspruch 1, **dadurch gekennzeichnet, dass** das Lösungsmittelgemisch aus Alkohol und n-Hexan in einem vol./vol.-Verhältnis von 1 : 2 in eine Mischung von RRRS, SSSR, RRSR und SSRS-Konfigurationen von Nebivolov-Zwischenprodukten gemäß Formel I gegeben wird, dann die Lösung zum Rückfluss erhitzt wird und auf 10 - 15°C abgekühlt wird und nach Kristallisieren für 4 Stunden und Filtern das Produkt erhalten werden kann.

## Revendications

1. Procédé pour la préparation d'un mélange de configurations RRRS et SSSR d'intermédiaires du nébivolol représentés par les formules II et III respectivement, **caractérisé en ce que** : une des deux façons suivantes soit optionnellement choisie :
(1) au solvant alcoolique comprenant le mélange de configurations RRRS, SSSR, RRSR et SSRS d'intermédiaires du nébivolol représentés par la formule I, est ajouté le solvant de précipitation : après chauffage, refroidissement pour précipiter des cristaux et filtration, le produit peut être obtenu ;
(2) le mélange de configurations RRRS, SSSR, RRSR et SSRS d'intermédiaires du nébivolol représentés par la formule I est ajouté dans le solvant mixte de solvant alcoolique et de solvant de précipitation : après chauffage, refroidissement pour précipiter des cristaux et filtration, le produit peut être obtenu ; 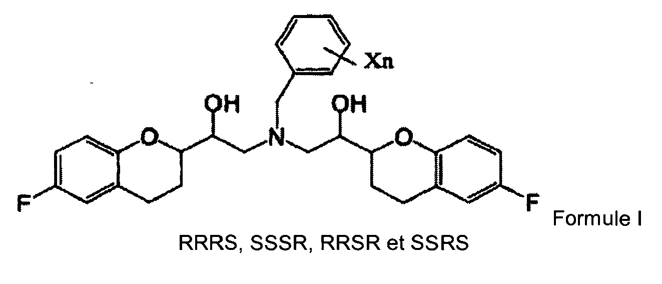 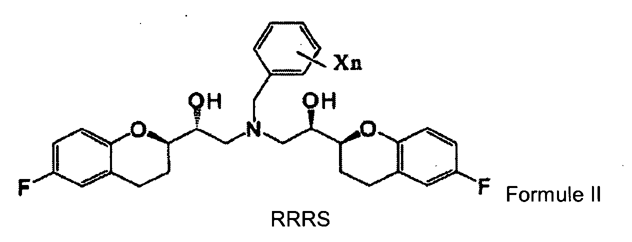 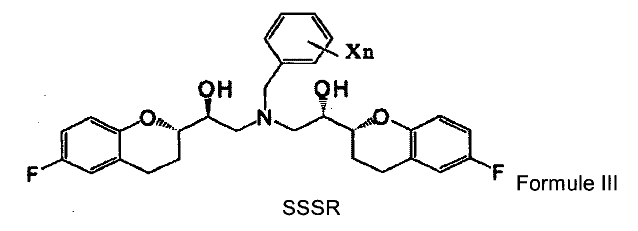
où X est H, alkyle en C₁ à C₆ ou alcoxy en C₁ à C₆, n est compris entre 1 et 5 et où ledit solvant de précipitation est un solvant d'hydrocarbure et/ou un solvant de type éther.

2. Procédé selon la revendication 1, **caractérisé en ce que** : ledit solvant alcoolique est un ou plusieurs choisis parmi le groupe constitué de méthanol, éthanol, propanol et alcool isopropylique.

3. Procédé selon la revendication 2, **caractérisé en ce que** : ledit solvant alcoolique est l'éthanol.

4. Procédé selon la revendication 1, **caractérisé en ce que** : lesdits solvants hydrocarbures sont des solvants hydrocarbures en C₅ à C₉ ; ledit solvant de type éther est l'éther isopropylique.

5. Procédé selon la revendication 4, **caractérisé en ce que** : ledit solvant d'hydrocarbure en C₅ à C₉ est un ou plusieurs choisis parmi le groupe constitué de n-hexane, n-heptane, éther de pétrole et cyclohexane.

6. Procédé selon la revendication 5, **caractérisé en ce que** : ledit solvant d'hydrocarbure en C₅ à C₉ est le n-hexane.

7. Procédé selon la revendication 1, **caractérisé en ce que** : le rapport en volume dudit solvant de précipitation et dudit solvant alcoolique est 1/2 - 4/1.

8. Procédé selon la revendication 7, **caractérisé en ce que** : le rapport en volume dudit solvant de précipitation et dudit solvant alcoolique est 2/1.

9. Procédé selon la revendication 1, **caractérisé en ce que** : la température dudit chauffage est 30 - 80 °C.

10. Procédé selon la revendication 9, **caractérisé en ce que** : la température dudit chauffage est la température de reflux des solvants.

11. Procédé selon la revendication 1, **caractérisé en ce que** : la température dudit refroidissement est 0 ~ 50 °C.

12. Procédé selon la revendication 11, **caractérisé en ce que** : la température dudit refroidissement est 10 ~ 15 °C.

13. Procédé selon la revendication 1, **caractérisé en ce que** : ladite durée de précipitation des cristaux est 2 - 18 heures.

14. Procédé selon la revendication 1, **caractérisé en ce que** : à la solution alcoolique qui contient le mélange de configurations RRRS, SSSR, RRSR et SSRS d'intermédiaire du nébivolol représenté par la formule I, est ajouté du n-hexane en double du volume d'éthanol, puis la solution est chauffée à reflux et refroidie à 10 ~ 15 °C, après cristallisation pendant 4 heures et filtration, le produit peut être obtenu.

15. Procédé selon la revendication 1, **caractérisé en ce que** : le solvant mixte d'alcool et de n-hexane avec un rapport v/v de 1/2 est ajouté dans le mélange de configurations RRRS, SSSR, RRSR et SSRS d'intermédiaire du nébivolol représenté par la formule I, puis la solution est chauffée à reflux, et refroidie à 10 ~ 15 °C ; après cristallisation pendant 4 heures et filtration, le produit peut être obtenu.
